# EUROPEAN PATENT SPECIFICATION

(11) **EP 4 642 531 B1**
(45) Date of publication and mention of the grant of the patent: **07.10.2026**
(21) Application number: 23837572.9
(22) Date of filing: 20.12.2023
(51) Int. Cl.: A61N 1/39

(54) **DEFIBRILLATION CONTROLLER ADVISING ABOUT SHOCK IMPEDANCE PRIOR TO A SHOCK DELIVERY**
DEFIBRILLATIONSSTEUERGERÄT, DAS VOR DER ABGABE EINES SCHOCKS ÜBER DIE SCHOCKIMPEDANZ INFORMIERT
CONTRÔLEUR DE DÉFIBRILLATION INFORMANT DE L'IMPÉDANCE DE CHOC AVANT L'ADMINISTRATION D'UN CHOC

(30) Priority: 31.12.2022 US 202263436554 P
(43) Date of publication of application: 05.11.2025
(73) Proprietor: Everest Acquisition Entity, LLC, Wilmington, DE 19805 (US)
(72) Inventor: GEHMAN, Stacy Earl, 5656 AG Eindhoven (NL); LIU, Chenguang, 5656 AG Eindhoven (NL); JORGENSON, Dawn Blilie, 5656 AG Eindhoven (NL)
(74) Representative: Weilnau, Carsten
(86) International application number: PCT/EP2023/086810
(87) International publication number: WO 2024/141359

(56) References cited:
- US-A1- 2015 112 401
- US-A1- 2017 106 198
- US-A1- 2017 361 119
- US-A1- 2022 193 433
- US-B2- 8 532 765
- US-B2- 9 757 581
- US-B2- 9 889 313

## Description

### FIELD OF THE INVENTION

The present disclosure generally relates to a cardiac arrest treatment involving a defibrillation of a heart of a patient and more specifically related to improving a defibrillation of a heart of a patient by a defibrillation discharge circuit associated with the defibrillator.

### BACKGROUND OF THE INVENTION

US2022193433A1 discloses a system for detecting and addressing irregular motion to improve defibrillation shock recommendations.

US2017106198A1 discloses a wearable cardiac defibrillator system with impedance measurement circuit to control conductive fluid deployment.

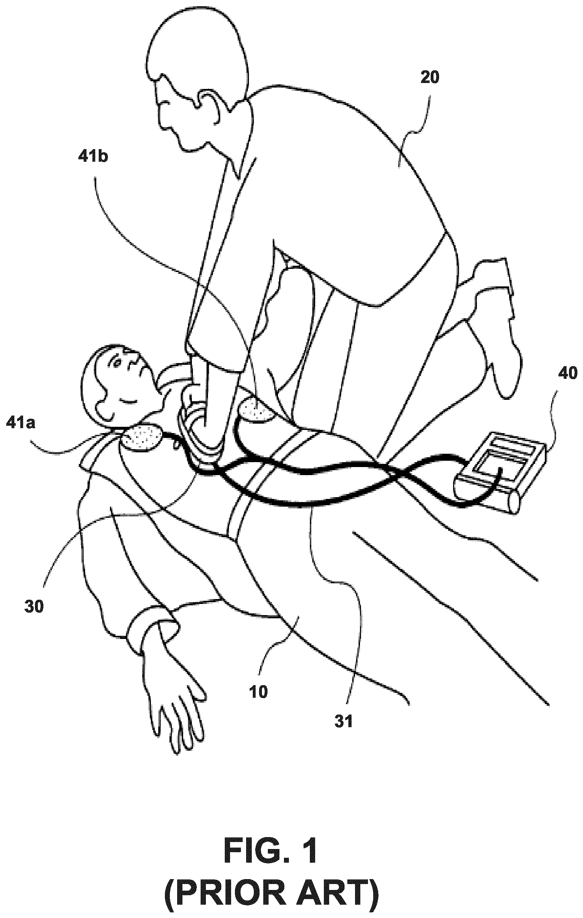

FIG. 1 illustrates a CPR monitor 30 positioned on the sternum of a patient 10 as a responder 20 applies chest compressions in a conventional manner using two hands with one placed over the other. Instead of placing the hands directly on the patient 10, however, the hands of responder 20 are placed on the CPR monitor 30 and chest compressions are applied to the patient 10 via the CPR monitor 30. Chest compressions are administered by the responder 20 to a heart of patient 10 as prescribed by conventional CPR protocols. As known in the art of the present disclosure, the CPR monitor 30 monitors a quality of the CPR being administered by a responder 20 to a heart of patient 10, such as, for example, whether the CPR is effective or ineffective in terms of a depth and a rate of compression, chest release and recoil, and placement of the responder's hands on the chest of patient 10. A cable 31 is attached to a defibrillator 40 to couple the monitoring of the CPR quality to defibrillator 40 and to issue audible CPR instructions through a loudspeaker of defibrillator 40.

FIG. 1 further illustrates defibrillator 40 attached to patient 10 by electrodes 41a and 41b. Defibrillator 40, as known in the art of the present disclosure, is used to deliver defibrillating shocks to the patient 10 during the CPR as needed. More specifically, defibrillator 40 is operable to deliver a high-voltage impulse to a heart of patient 10 in order to restore normal rhythm and contractile function in patients who are experiencing an arrhythmia (e.g., ventricular fibrillation (VF) or ventricular tachycardia (VT)) that is not accompanied by spontaneous circulation. In operation, defibrillator 20 automatically analyzes an electrocardiogram (ECG) rhythm of the heart of patient 10 to determine if defibrillation is necessary. If so, defibrillator 40 prompts responder 20 to terminate the CPR and to press a shock button to deliver the defibrillation shock to the patient when a shock is advised by defibrillator 40.

The field of resuscitation, as exemplarily shown in FIG. 1, is heavily focused on increasing a quality of care by identifying and providing optimal CPR/shock treatment for a patient experiencing cardiac arrest.

### SUMMARY OF THE INVENTION

The present disclosure is directed to an improvement to existing defibrillators (e.g., Automated External Defibrillators and Advanced Life Support Defibrillators) by providing low shock impedance advisory when an estimated pre-shock impedance of a defibrillation discharge circuit associated with a defibrillator is greater than a shock impedance threshold.

The invention is defined by the independent claims. The dependent claims define advantageous embodiments. The present disclosure may be embodied as (1) a defibrillator controller and (2) a computer program product having instructions for implementing a defibrillation method.

Various exemplary embodiments of a defibrillator of the present disclosure encompass an ECG analyzer and a shock impedance advisor for improving a defibrillation of a heart of a patient by the defibrillation discharge circuit associated with the defibrillator. The ECG analyzer is configured to derive a shock delivery decision from a detection of a shockable rhythm in an ECG waveform of the patient. In response to the shock delivery decision, the shock impedance advisor is configured to (1) measure a non-shock impedance of the defibrillator discharge circuit, (2) estimate a pre-shock impedance of the defibrillator discharge circuit from a measurement of the non-shock impedance of the defibrillator discharge circuit, and (3) communicate a lower shock impedance advisory to a responder operating the defibrillator when the pre-shock impedance of the defibrillator discharge circuit is greater than a shock impedance threshold.

Various additional exemplary embodiments of a defibrillation controller of the present disclosure encompass a non-transitory machine-readable storage medium encoded with instructions for execution by one or more processors to improve a defibrillation of a heart of a patient by a defibrillation discharge circuit associated with a defibrillator. In response to the shock delivery decision, the non-transitory machine-readable storage medium includes instructions to (1) measure a non-shock impedance of the defibrillator discharge circuit, (2) estimate a pre-shock impedance of the defibrillator discharge circuit from a measurement of the non-shock impedance of the defibrillator discharge circuit, and (3) communicate a lower shock impedance advisory to a responder operating the defibrillator when the pre-shock impedance of the defibrillator discharge circuit is greater than a shock impedance threshold.

Various exemplary embodiments of a defibrillation method implemented by a defibrillation controller programmed by instructions of a computer program product in accordance with the present disclosure encompass improving a defibrillation of a heart of a patient by the defibrillation discharge circuit associated with the defibrillator. The defibrillation method involves a defibrillator deriving a shock delivery decision from a detection of a shockable rhythm in an ECG waveform of the patient. In response to the shock delivery decision, the defibrillation method further involves the defibrillator (1) measuring a non-shock impedance of the defibrillator discharge circuit, (2) estimating a pre-shock impedance of the defibrillator discharge circuit from a measurement of the non-shock impedance of the defibrillator discharge circuit, and (3) communicating a lower shock impedance advisory to a responder operating the defibrillator when the pre-shock impedance of the defibrillator discharge circuit is greater than a shock impedance threshold.

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1 illustrates a cardiopulmonary resuscitation being administered by a responder to a heart of the patient as known in the art of the present disclosure;
FIG. 2 illustrates an exemplary embodiment of a cardiac arrest treatment system in accordance with the present disclosure;
FIG. 3 illustrates an exemplary embodiment of a defibrillation discharge circuit in accordance with the present disclosure;
FIG. 4 illustrates an exemplary embodiment of a defibrillator in accordance with the present disclosure;
FIG. 5 illustrates a flowchart representative of an exemplary embodiment of a defibrillation method in accordance with the present disclosure; and
FIG. 6 illustrates an exemplary embodiment of a defibrillation controller in accordance with the present disclosure.

### DETAILED DESCRIPTION OF THE PREFERRED EMBODIMENTS

The present disclosure is directed to an improvement to existing defibrillators (e.g., Automated External Defibrillators and Advanced Life Support Defibrillators) by providing termination of resuscitation (TOR) advisory in support a TOR decision by a responder during an administration of the cardiopulmonary resuscitation (CPR) by the responder to a heart of a patient.

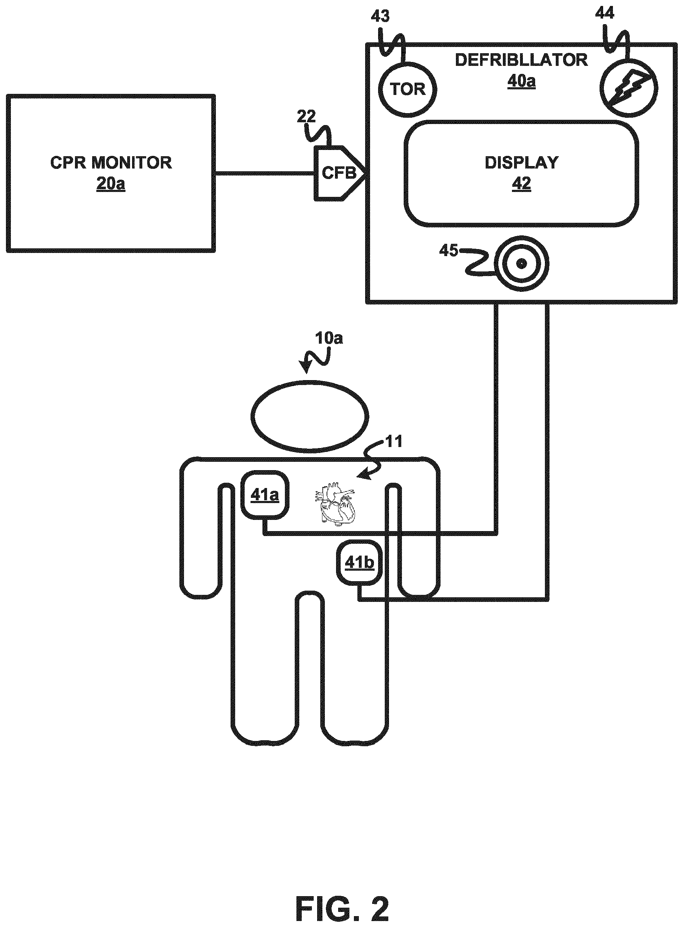

To facilitate an understanding of the present disclosure, the following description of FIG. 2 teaches an exemplary embodiment of a cardiac arrest treatment system in accordance with the present disclosure. From the description of FIG. 2, those having ordinary skill in the art of the present disclosure will appreciate how to apply the present disclosure to make and use additional embodiments of cardiac arrest treatment systems in accordance with the present disclosure.

Referring to FIG. 2, the exemplary cardiac arrest treatment system of the present disclosure employs a CPR monitor 20a and a defibrillator 40a.

In practice, CPR monitor 20a is any device, as known in the art of the present disclosure or hereinafter conceived, for analyzing a quality of CPR being administered by a responder (not shown) to a heart 11 of a patient 10a.

In a first exemplary embodiment, CPR monitor 20a is configured as a CPR coaching device in accordance with US 8,532,765 B2 entitled "CPR Coaching Device with Reduced Sensitivity to Motion" and mentioning *Ochs* et al. as inventors.

In a second exemplary embodiment, monitor 20a is a CPR mechanical device incorporating the CPR analyzing principles described in *Ochs* et al.

Still referring to FIG. 2, in practice, defibrillator 40a is any type of defibrillator, as known in the art of the present disclosure or hereinafter conceived, incorporating the inventive principles of the present disclosure for support a TOR decision by a responder during an administration of the cardiopulmonary resuscitation (CPR) by the responder to a heart of a patient.

In operation, defibrillator 40a inputs a CPR feedback 22 from CPR monitor 20a as a basis for monitoring the administration of the CPR by the responder on the heart of the patient.

Further in operation, defibrillator 40a monitors an ECG waveform of heart 11 of patient 10a via electrode 41a and 41b applied to patient 10a as known in the art of the present disclosure, and analyzes ECG waveform to detect any shockable rhythm of heart 11 of patient 10a as known in the art of the present disclosure.

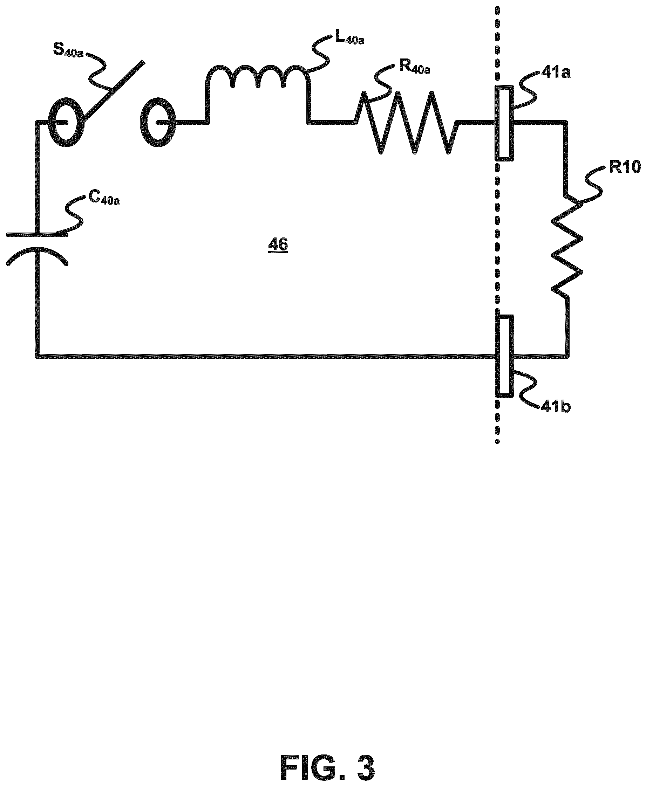

If defibrillator 40a derives a shockable delivery decision from an analysis of the ECG waveform, then defibrillator 40a will measure a non-shock impedance of a defibrillation discharge circuit 46 as shown in FIG. 3 as a basis for estimating if a pre-shock impedance of the defibrillation discharge circuit 46 is above a shock defibrillation threshold as will be further described in the present disclosure.

Referring to FIG. 3, defibrillation discharge circuit 46 includes a capacitor C40a, a switch S40a, an inductor L40a and a resistor R40a of defibrillator 40a, and further includes a resistor R10 representative of an impedance of patient 10.

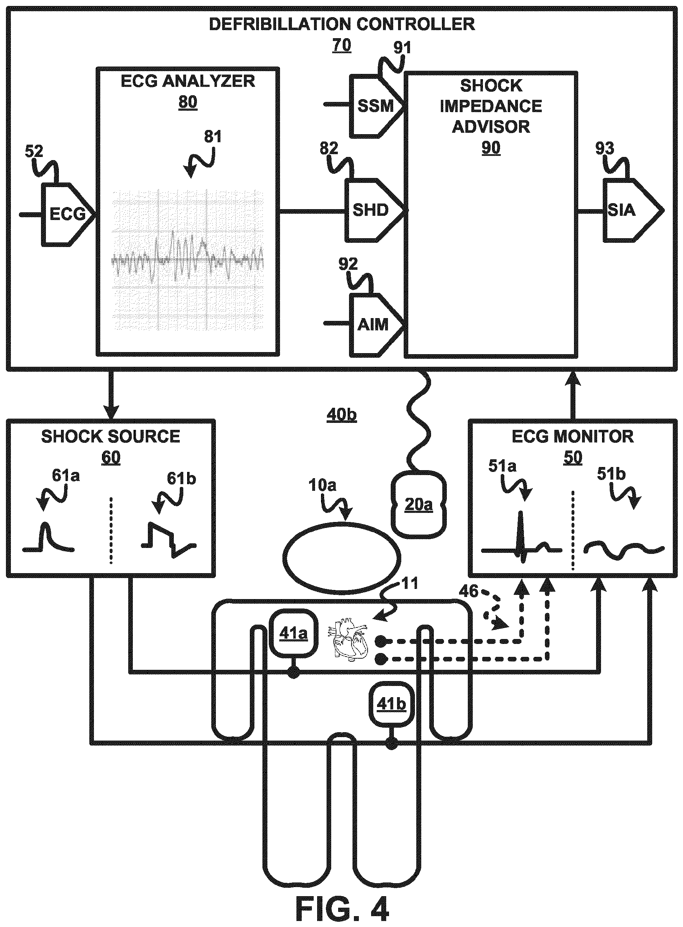

To further facilitate an understanding of the present disclosure, the following description of FIG. 4 teaches an exemplary embodiment of a defibrillator in accordance with the present disclosure. From the description of FIG. 4, those having ordinary skill in the art of the present disclosure will appreciate how to apply the present disclosure to make and use additional embodiments of defibrillator in accordance with the present disclosure.

Referring to FIG. 4, a defibrillator 40b of the present disclosure employs the pair of electrode pads/paddles 41a and 41b, optional ECG leads 46, an ECG monitor 50 (internal or external), a shock source 60, a defibrillation controller 70. Also shown is a CPR coaching device 20a communicatively coupled to defibrillation controller 70.

Electrode pads/paddles 41a and 41b are structurally configured as known in the art of the present disclosure to be conductively applied to a patient 10a in an anterior-apex arrangement as shown in FIG. 1 or alternatively in an anterior-posterior arrangement (not shown). Electrode pads/paddles 41a and 41b conduct a defibrillation shock from shock source 60 to heart 11 of patient 10a as controlled by defibrillation controller70 as known in the art of the present disclosure, and conduct electrical activity of heart 11 of patient 10a to ECG monitor 50 as known in the art of the present disclosure. Alternatively or concurrently, ECG leads 46 as known in the art of the present disclosure may be connected to patient 10a to conduct the electrical activity of heart 11 of patient 10a to ECG monitor 50.

ECG monitor 50 is structurally configured as known in the art to generate an ECG waveform of heart 11 of patient 10a as an indication patient 10a is experiencing an organized heartbeat condition or an unorganized heartbeat condition. An example of ECG waveform indicating an organized heartbeat condition is an ECG waveform 51a as shown in FIG. 3 that is representative of an organized contraction of the ventricles of heart 11 being capable of pumping blood. An example of ECG waveform indicating patient 10a is experiencing an unorganized heartbeat condition is a random ECG waveform 51b as shown in FIG. 3 having zero (0) discernible waves representative of no organized heartbeat activity of heart 11 of patient 10a.

In one exemplary embodiment, ECG monitor 50 employs a digital signal processor (not shown) for streaming ECG waveform data 52 to defibrillation controller 70.

Shock source 60 is structurally configured as known in the art of the present disclosure to store electric energy for delivery of a defibrillation shock via electrode pads/paddles 41a and 41b to heart 11 of patient 10a as controlled by defibrillation controller 70. In practice, the defibrillation shock may have any waveform as known in the art of the present disclosure. Examples of such waveforms include, but are not limited to, a monophasic sinusoidal waveform (positive sine wave) 61a and a biphasic truncated waveform 61b as shown in FIG. 3.

In one exemplary embodiment, shock source 60 employs a high voltage capacitor bank (not shown) for storing a high voltage via a high voltage charger and a power supply upon a pressing of a charge button. Shock source 60 further employs a switching/isolation circuit (not shown) for selectively applying a specific waveform of an electric energy charge from the high voltage capacitor bank to electrode pads/paddles 41a and 41b as controlled by defibrillation controller 70.

Defibrillation controller 70 incorporates an ECG analyzer 80, as known in the art of the present disclosure and hereinafter conceived, for analyzing and interpreting ECG waveform data 52 from ECG monitor 50.

Defibrillation controller 70 further incorporates a shock impedance advisor 90 for conditionally deriving and communicating a low shock impedance advisory 91 to the responder.

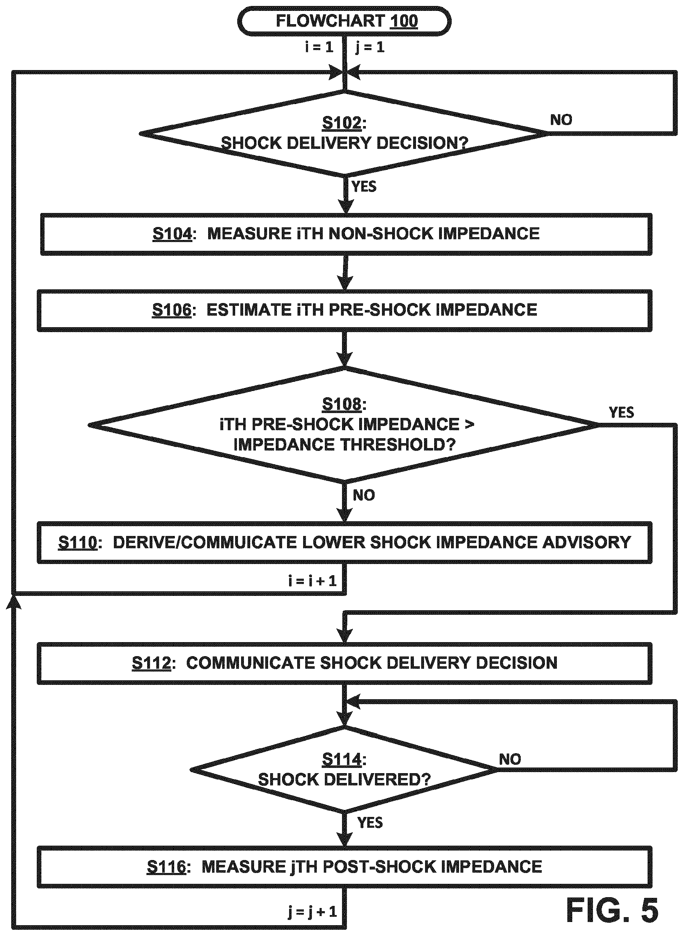

In one embodiment, a flowchart 100 as shown in FIG. 5 is executed by shock impedance advisor 90 upon a commencement of CPR by the responder.

Referring to FIG. 5, in response to receiving a shock delivery decision at a stage S102 of flowchart 100, shock impedance advisor 90 proceeds to a stage S104 to measure a non-shock impedance of the defibrillation discharge circuit 46 (FIG. 3) and to a stage S105 to estimate a pre-shock impedance of defibrillation discharge circuit 46.

In one embodiment of stage S104, shock impedance advisor 90 delivers a small signal to defibrillation discharge circuit 46 using 32kHz or 12.8KHz, for example at an energy level to low to induce a shock of the heart of patient 10, and measures the non-shock impedance of defibrillation discharge circuit 46 as known in the art of the present disclosure.

In one embodiment of stage S104, shock impedance advisor 90 implements a supervised learning model trained on a correlation of pre-shock impedances of defibrillation discharge circuit 46 to non-shock impedances of defibrillation discharge circuit 46 to thereby estimate the pre-shock impedance from the measured non-shock impedance.

If the pre-shock impedance is greater than a shock impedance threshold (e.g., 80-100 ohms) at stage S108 of flowchart 100, then shock impedance advisor 90 will derive and communicate lower shock impedance advisory 93 at stage S110 of flowchart 100 and return to stage S102. In one embodiment, lower shock impedance advisory 93 may instruct a changing of the pads' placement on the patient's chest, the responder can change the configuration of the pads from Anterior/Anterior (A/A) to Anterior/Posterior (A/P). Another action the responder can take is to press down on the pads to ensure they are adhered firmly to the patient and reassess the shock impedance. A new defibrillation vector with a lower estimated shock impedance can provide more current and potentially an improved defibrillation shock.

If the pre-shock impedance is less than a shock impedance threshold (e.g., 80-100 ohms) at stage S108 of flowchart 100, then shock impedance advisor 90 will communicate the shock delivery decision at a S112 of flowchart 100. Upon delivery of the shock at stage S114, then shock impedance advisor 90 will proceed to measure a post-shock impedance of the defibrillation discharge circuit and return to stage S102.

In practice, it is possible for this estimated shock impedance to be conveyed to the responder by a visual indicator (e.g., number, color, symbol, etc.) and/or audio (e.g., simulated voice, sound pitches, alerts, etc.).

In accordance with exemplary embodiments of the present disclosure, the non-shock impedance can be used to estimate shock impedance before a shock is delivered. The actual shock impedance can be measured when a shock is delivered. Both the estimated and real shock impedance can be used for feedback to indicate to the responder that the impedance is (very) high or (very) low.

As provided above, the non-shock impedance can be useful before any shocks are administered (e.g., to prompt the responder to take action to lower the impedance before administering a shock). The combined impedance determination (estimated and actual measured) can be used after a shock has been delivered to inform and assist the responder in taking action to lower the impedance before another shock is advised and administered/delivered.

The actual measured impedance can also be used to alert the responder of a (significant) variance/difference between the estimated impedance (e.g., based on small signal) and the actual impedance (i.e., based on impedance measured during shock delivery), and adjust for a future estimated impedance (e.g., by adjusting the small signal used and/or adjusting the estimated results after the small measurement is taken based on the actual impedance and/or combined impedance measurements (i.e., based on both the estimated and real/actual shock impedance) and/or address other factors that can affect the estimated shock impedance measurement).

The average shock impedance typically should be 80-100 ohms. The success rate of defibrillation may be lower if the patient's shock impedance is (very) high. Responders can use paddles or otherwise press on the patient's thorax with a rolled towel, for example, to (1) squeeze air out of chest and (2) increase adherence of the pads to the chest of the patient in an effort to lower shock impedance.

Shock impedance is mostly important if/when a patient has a shockable rhythm. If/when the patient has a non-shockable rhythm, then the exemplary device, system and/or method can still analyze the patient's impedance while continuing to monitor the patient's ECG and cardiac rhythm. Even during times when a non-shockable rhythm is detected, exemplary embodiments of the present disclosure can still alert and communicate to the responder that the impedance is high and certain actions should be considered (e.g., a pad position change) so that the responder can take appropriate action if and before a shockable rhythm is detected and a shock is advised and administered/ delivered.

For example, an action taken by the responder if the shock impedance is high can be to try another pad configuration. In addition to changing the pads placement on the patient's chest, the responder can change the placement of the pads from Anterior/Anterior (A/A) to Anterior/Posterior (A/P) and reassess the estimated shock impedance. Another action the responder can take is to press down on the pads to ensure they are adhered firmly to the patient and reassess the shock impedance. A new defibrillation vector with a lower estimated shock impedance can provide more current and potentially an improved defibrillation shock.

Exemplary embodiments of the present disclosure can also be used with/for Duel Sequential Defibrillation (DSD) as would be appreciated by those having ordinary skill in the art, which uses multiple shocks given in quick succession from one or more defibrillators at different defibrillation vectors. The use of estimated shock impedance and/or combined impedance measurements (i.e., based on both the estimated and real shock impedance) in accordance with exemplary embodiments of the present disclosure can help to improve and identify/estimate the likely best and/or optimal (or near best/optimal) defibrillation vectors for future shocks.

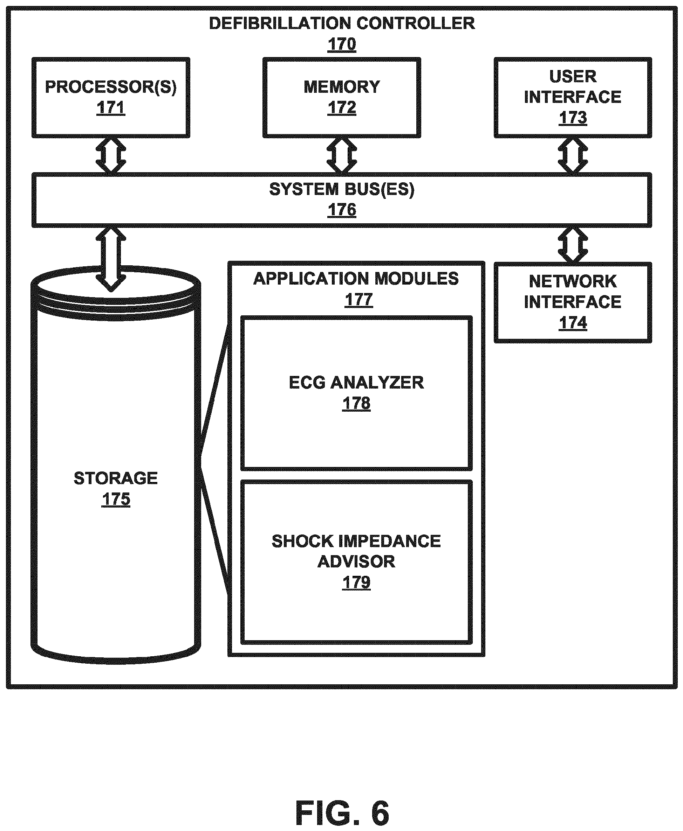

To facilitate a further understanding of the present disclosure, the following description of FIG. 6 teaches an exemplary embodiment of defibrillation controller in accordance with the present disclosure. From the description of FIG. 6, those having ordinary skill in the art of the present disclosure will appreciate how to apply the present disclosure to make and use additional embodiments of a defibrillation controller in accordance with the present disclosure.

Referring to FIG. 5, shown is an exemplary embodiment of defibrillation controller 170 that includes one or more processor(s) 171, memory 172, a user interface 173, a network interface 174, and a storage 175 interconnected via one or more system bus(es) 176.

Each processor 171 can be any hardware device, as known in the art of the present disclosure or hereinafter conceived, capable of executing instructions stored in memory 172 or storage or otherwise processing data. In a non-limiting example, the processor(s) 171 can include a microprocessor, field programmable gate array (FPGA), application-specific integrated circuit (ASIC), or other similar devices.

The memory 172 can include various memories, as known in the art of the present disclosure or hereinafter conceived, including, but not limited to, L1, L2, or L3 cache or system memory. In a non-limiting example, the memory 172 can include static random access memory (SRAM), dynamic RAM (DRAM), flash memory, read only memory (ROM), or other similar memory devices.

The user interface 173 can include one or more devices, as known in the art of the present disclosure or hereinafter conceived, for enabling communication with a user such as an administrator. In a non-limiting example, the user interface can include a command line interface or graphical user interface that can be presented to a remote terminal via the network interface 174.

The network interface 174 can include one or more devices, as known in the art of the present disclosure or hereinafter conceived, for enabling communication other components of a medical device. In a non-limiting example, the network interface 174 can include a network interface card (NIC) configured to communicate according to the Ethernet protocol. Additionally, the network interface 174 may implement a TCP/IP stack for communication according to the TCP/IP protocols. Various alternative or additional hardware or configurations for the network interface 174 will be apparent.

The storage 175 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 175 can store instructions for execution by the processor(s) 171 or data upon with the processor(s) 171 may operate. For example, the storage 175 may store a base operating system for controlling various basic operations of the hardware.

The storage 175 can also store an application modules in the form of executable software/firmware for implementing the various functions of the methods of FIGS. 4 and 6 as previously described in the present disclosure.

In one exemplary embodiment as shown, storage 175 stores application modules 177 including an ECG analyzer 178 for deriving shock delivery decisions as known in the art of the present disclosure and shock impedance advisor 170 for deriving a low shock impedance advisory as previously described in the present disclosure, particularly in accordance with flowchart 100 of FIG. 5.

From the description of FIGS. 1-6 herein, those having ordinary skill in the art will appreciate the numerous benefits of the present disclosure including, but not limited to, improving a defibrillation of a heart of a patient by the defibrillation discharge circuit associated with a defibrillator.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which may be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer-readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

## Claims

1. A defibrillation controller (70) for improving a defibrillation of a heart of a patient by a defibrillation discharge circuit (46) associated with a defibrillator (40), the defibrillation controller comprising:
an ECG analyzer (80) configured to derive a shock delivery decision from a detection of a shockable rhythm in an ECG waveform of the patient; and
a shock impedance advisor (90) **characterized in that** the shock impedance advisor is configured, in response to the shock delivery decision, to:
measure a non-shock impedance of the defibrillator discharge circuit;
estimate a pre-shock impedance of the defibrillator discharge circuit from a measurement of the non-shock impedance of the defibrillator discharge circuit; and
communicate a lower shock impedance advisory to a responder operating the defibrillator when the pre-shock impedance of the defibrillator discharge circuit is greater than a shock impedance threshold.

2. The defibrillation controller of claim 2, wherein the shock impedance advisor (90) is further configured to:
communicate the shock delivery decision to the responder operating the defibrillator (40) when the pre-shock impedance of the defibrillator discharge circuit (46) is less than the shock impedance threshold.

3. The defibrillation controller of claim 2,
wherein the shock impedance advisor (90) is further configured, in response to a shock delivery by the defibrillator (40), to measure a post-shock impedance of the defibrillator discharge circuit (46); and
wherein the shock impedance advisor (90) is further configured, in response to a second shock delivery decision by ECG analyzer (80) subsequent to the shock delivery by the defibrillator (40), to:
measure a non-shock impedance of a defibrillator discharge circuit (46);
estimate a second pre-shock impedance of the defibrillator discharge circuit (46) from a measurement of the second non-shock impedance of the defibrillator discharge circuit and a measurement of the post-shock impedance of the defibrillator discharge circuit; and
communicate a second lower shock impedance advisory to a responder operating the defibrillator (40) when the second pre-shock impedance of the defibrillator of the defibrillator discharge circuit (46) is greater than the shock impedance threshold.

4. The defibrillation controller of claim 3, wherein the shock impedance advisor (90) is further configured to:
communicate the second shock delivery decision to the responder operating the defibrillator (40) when the second pre-shock impedance of the defibrillator discharge circuit (46) is less than the shock impedance threshold.

5. The defibrillation controller of claim 1, wherein the shock delivery decision is for one of a monophasic sinusoidal defibrillation of the heart of the patient or a biphasic truncated waveform of the heart of the patient.

6. A computer program product comprising instructions for execution by at least one processor to improve a defibrillation of a heart of a patient by a defibrillation discharge circuit associated with a defibrillator,
**characterized in that**, in response to a shock delivery decision, the computer program product includes instructions to:
measure a non-shock impedance of the defibrillator discharge circuit;
estimate a pre-shock impedance of the defibrillator discharge circuit from a measurement of the non-shock impedance of the defibrillator discharge circuit; and
communicate a lower shock impedance advisory to a responder operating the defibrillator when the pre-shock impedance of the defibrillator discharge circuit is greater than a shock impedance threshold.

7. The computer program product of claim 6, wherein, in response to the shock delivery decision, the computer program product further includes instructions to:
communicate the shock delivery decision to the responder operating the defibrillator when the pre-shock impedance of the defibrillator discharge circuit is less than the shock impedance threshold.

8. The computer program product of claim 7,
wherein in response to a shock delivery by the defibrillator, the computer program product further includes instructions to:
measure a post-shock impedance of the defibrillator discharge circuit; and
wherein, in response to a second shock delivery decision by ECG analyzer subsequent to the shock delivery by the defibrillator, the computer program product further includes instructions to:
measure a non-shock impedance of a defibrillator discharge circuit;
estimate a second pre-shock impedance of the defibrillator discharge circuit from a measurement of the second non-shock impedance of the defibrillator discharge circuit and a measurement of the post-shock impedance of the defibrillator discharge circuit; and
communicate a second lower shock impedance advisory to a responder operating the defibrillator when the second pre-shock impedance of the defibrillator of the defibrillator discharge circuit is greater than the shock impedance threshold.

9. The computer program product of claim 6, wherein, in response to a second shock delivery decision by ECG analyzer subsequent to the shock delivery by the defibrillator, the computer program product further includes instructions to:
communicate the second shock delivery decision to the responder operating the defibrillator when the second pre-shock impedance of the defibrillator discharge circuit is less than the shock impedance threshold.

10. The computer program product of claim 6, wherein the shock delivery decision is for one of a monophasic sinusoidal defibrillation of the heart of the patient or a biphasic truncated waveform of the heart of the patient.

11. A defibrillator (40b), comprising:
an ECG monitor (50) configured to generate an ECG waveform of heart (11) of patient (10a); and
a defibrillator controller (70) as claimed in any of claims 1-5.

## Patentansprüche

1. Defibrillationssteuerung (70) zum Verbessern einer Defibrillation eines Herzens eines Patienten durch eine Defibrillations-Entladeschaltung (46), die mit einem Defibrillator (40) verbunden ist, wobei die Defibrillationssteuerung umfasst:
einen EKG-Analysator (80), der dazu ausgelegt ist, eine Schockabgabeentscheidung aus einer Detektion eines schockbaren Rhythmus in einer EKG-Wellenform des Patienten abzuleiten; und
einen Schockimpedanzassistenten (90), **dadurch gekennzeichnet, dass** der Schockimpedanzassistent als Reaktion auf die Schockabgabeentscheidung ausgelegt ist zum:
Messen einer Nicht-Schock-Impedanz der Defibrillator-Entladeschaltung;
Schätzen einer Vor-Schock-Impedanz der Defibrillator-Entladeschaltung anhand einer Messung der Nicht-Schock-Impedanz der Defibrillator-Entladeschaltung; und
Übermitteln einer Empfehlung für eine niedrigere Schockimpedanz an einen Ersthelfer, der den Defibrillator betätigt, wenn die Vor-Schock-Impedanz der Defibrillator-Entladeschaltung größer als ein Schockimpedanz-Schwellenwert ist.

2. Defibrillationssteuerung nach Anspruch 2, wobei der Schockimpedanzassistent (90) ferner ausgelegt ist zum:
Übermitteln der Schockabgabeentscheidung an den Ersthelfer, der den Defibrillator (40) betätigt, wenn die Vor-Schock-Impedanz der Defibrillator-Entladeschaltung (46) kleiner als der Schockimpedanz-Schwellenwert ist.

3. Defibrillationssteuerung nach Anspruch 2,
wobei der Schockimpedanzassistent (90) ferner dazu ausgelegt ist, als Reaktion auf eine Schockabgabe durch den Defibrillator (40) eine Nach-Schock-Impedanz der Defibrillator-Entladeschaltung (46) zu messen; und wobei der Schockimpedanzassistent (90) ferner als Reaktion auf eine zweite Schockabgabeentscheidung durch den EKG-Analysator (80) nach der Schockabgabe durch den Defibrillator (40) ausgelegt ist zum:
Messen einer Nicht-Schock-Impedanz einer Defibrillator-Entladeschaltung (46);
Schätzen einer zweiten Vor-Schock-Impedanz der Defibrillator-Entladeschaltung (46) anhand einer Messung der zweiten Nicht-Schock-Impedanz der Defibrillator-Entladeschaltung und einer Messung der Nach-Schock-Impedanz der Defibrillator-Entladeschaltung; und
Übermitteln einer zweiten Empfehlung für eine niedrigere Schockimpedanz an einen Ersthelfer, der den Defibrillator (40) betätigt, wenn die zweite Vor-Schock-Impedanz des Defibrillators der Defibrillator-Entladeschaltung (46) größer als der Schockimpedanz-Schwellenwert ist.

4. Defibrillationssteuerung nach Anspruch 3, wobei der Schockimpedanzassistent (90) ferner ausgelegt ist zum: Übermitteln der zweiten Schockabgabeentscheidung an den Ersthelfer, der den Defibrillator (40) betätigt, wenn die zweite Vor-Schock-Impedanz der Defibrillator-Entladeschaltung (46) kleiner als der Schockimpedanz-Schwellenwert ist.

5. Defibrillationssteuerung nach Anspruch 1, wobei die Schockabgabeentscheidung für eines von einer monophasischen sinusförmigen Defibrillation des Herzens des Patienten oder einer biphasischen trunkierten Wellenform des Herzens des Patienten ist.

6. Computerprogrammprodukt, das Anweisungen zur Ausführung durch mindestens einen Prozessor umfasst, um eine Defibrillation eines Herzens eines Patienten durch eine Defibrillations-Entladeschaltung zu verbessern, die mit einem Defibrillator verbunden ist,
**dadurch gekennzeichnet, dass** das Computerprogrammprodukt als Reaktion auf eine Schockabgabeentscheidung Anweisungen beinhaltet zum:
Messen einer Nicht-Schock-Impedanz der Defibrillator-Entladeschaltung;
Schätzen einer Vor-Schock-Impedanz der Defibrillator-Entladeschaltung anhand einer Messung der Nicht-Schock-Impedanz der Defibrillator-Entladeschaltung; und
Übermitteln einer Empfehlung für eine niedrigere Schockimpedanz an einen Ersthelfer, der den Defibrillator betätigt, wenn die Vor-Schock-Impedanz der Defibrillator-Entladeschaltung größer als ein Schockimpedanz-Schwellenwert ist.

7. Computerprogrammprodukt nach Anspruch 6, wobei das Computerprogrammprodukt als Reaktion auf die Schockabgabeentscheidung ferner Anweisungen beinhaltet zum:
Übermitteln der Schockabgabeentscheidung an den Ersthelfer, der den Defibrillator betätigt, wenn die Vor-Schock-Impedanz der Defibrillator-Entladeschaltung kleiner als der Schockimpedanz-Schwellenwert ist.

8. Computerprogrammprodukt nach Anspruch 7,
wobei das Computerprogrammprodukt als Reaktion auf eine Schockabgabe durch den Defibrillator ferner Anweisungen beinhaltet zum:
Messen einer Nach-Schock-Impedanz der Defibrillator-Entladeschaltung; und
wobei das Computerprogrammprodukt als Reaktion auf eine zweite Schockabgabeentscheidung durch den EKG-Analysator nach der Schockabgabe durch den Defibrillator ferner Anweisungen beinhaltet zum:
Messen einer Nicht-Schock-Impedanz einer Defibrillator-Entladeschaltung;
Schätzen einer zweiten Vor-Schock-Impedanz der Defibrillator-Entladeschaltung anhand einer Messung der zweiten Nicht-Schock-Impedanz der Defibrillator-Entladeschaltung und einer Messung der Nach-Schock-Impedanz der Defibrillator-Entladeschaltung; und Übermitteln einer zweiten Empfehlung für eine niedrigere Schockimpedanz an einen Ersthelfer, der den Defibrillator betätigt, wenn die zweite Vor-Schock-Impedanz des Defibrillators der Defibrillator-Entladeschaltung größer als der Schockimpedanz-Schwellenwert ist.

9. Computerprogrammprodukt nach Anspruch 6, wobei das Computerprogrammprodukt als Reaktion auf eine zweite Schockabgabeentscheidung durch den EKG-Analysator nach der Schockabgabe durch den Defibrillator ferner Anweisungen beinhaltet zum:
Übermitteln der zweiten Schockabgabeentscheidung an den Ersthelfer, der den Defibrillator betätigt, wenn die zweite Vor-Schock-Impedanz der Defibrillator-Entladeschaltung kleiner als der Schockimpedanz-Schwellenwert ist.

10. Computerprogrammprodukt nach Anspruch 6, wobei die Schockabgabeentscheidung für eines von einer monophasischen sinusförmigen Defibrillation des Herzens des Patienten oder einer biphasischen trunkierten Wellenform des Herzens des Patienten ist.

11. Defibrillator (40b), umfassend:
einen EKG-Monitor (50), der dazu ausgelegt ist, eine EKG-Wellenform des Herzens (11) des Patienten (10a) zu erzeugen; und
eine Defibrillatorsteuerung (70) nach einem der Ansprüche 1-5.

## Revendications

1. Dispositif de commande de défibrillation (70) permettant d'améliorer la défibrillation du cœur d'un patient par un circuit de décharge de défibrillation (46) associé à un défibrillateur (40), le dispositif de commande de défibrillation comprenant :
un analyseur d'ECG (80) configuré pour dériver une décision d'administration de choc à partir de la détection d'un rythme nécessitant un choc dans une forme d'onde d'ECG du patient ; et
un module-conseil d'impédance de choc (90) **caractérisé en ce que** le module-conseil d'impédance de choc est configuré, en réponse à la décision d'administration de choc, pour :
mesurer une impédance hors choc du circuit de décharge de défibrillateur ;
estimer une impédance avant choc du circuit de décharge de défibrillateur à partir d'une mesure de l'impédance hors choc du circuit de décharge de défibrillateur ; et
communiquer un avis d'impédance de choc inférieure à un intervenant faisant fonctionner le défibrillateur lorsque l'impédance avant choc du circuit de décharge de défibrillateur est supérieure à un seuil d'impédance de choc.

2. Dispositif de commande de défibrillation selon la revendication 2, dans lequel le module-conseil d'impédance de choc (90) est en outre configuré pour :
communiquer la décision d'administration de choc à l'intervenant faisant fonctionner le défibrillateur (40) lorsque l'impédance avant choc du circuit de décharge de défibrillateur (46) est inférieure au seuil d'impédance de choc.

3. Dispositif de commande de défibrillation selon la revendication 2,
dans lequel le module-conseil d'impédance de choc (90) est en outre configuré, en réponse à une administration de choc par le défibrillateur (40), pour mesurer une impédance après choc du circuit de décharge de défibrillateur (46) ; et
dans lequel le module-conseil d'impédance de choc (90) est en outre configuré, en réponse à une seconde décision d'administration de choc par l'analyseur ECG (80) après l'administration de choc par le défibrillateur (40), pour :
mesurer une impédance hors choc d'un circuit de décharge de défibrillateur (46) ;
estimer une seconde impédance avant choc du circuit de décharge du défibrillateur (46) à partir d'une mesure de la seconde impédance hors choc du circuit de décharge de défibrillateur et d'une mesure de l'impédance après choc du circuit de décharge de défibrillateur ; et
communiquer un second avis d'impédance de choc inférieure à un intervenant faisant fonctionner le défibrillateur (40) lorsque la seconde impédance avant choc du défibrillateur du circuit de décharge de défibrillateur (46) est supérieure au seuil d'impédance de choc.

4. Dispositif de commande de défibrillation selon la revendication 3, dans lequel le module-conseil d'impédance de choc (90) est en outre configuré pour : communiquer la seconde décision d'administration de choc à l'intervenant faisant fonctionner le défibrillateur (40) lorsque la seconde impédance avant choc du circuit de décharge de défibrillateur (46) est inférieure au seuil d'impédance de choc.

5. Dispositif de commande de défibrillation selon la revendication 1, dans lequel la décision d'administration d'un choc est pour une défibrillation sinusoïdale monophasique du cœur du patient et/ou une forme d'onde tronquée biphasique du cœur du patient.

6. Produit programme informatique comprenant des instructions permettant l'exécution par au moins un processeur pour améliorer la défibrillation du cœur d'un patient par un circuit de décharge de défibrillation associé à un défibrillateur,
**caractérisé en ce que**, en réponse à une décision d'administration de choc, le produit programme informatique comprend des instructions pour :
mesurer une impédance hors choc du circuit de décharge de défibrillateur ;
estimer une impédance avant choc du circuit de décharge de défibrillateur à partir d'une mesure de l'impédance hors choc du circuit de décharge de défibrillateur ; et
communiquer un avis d'impédance de choc inférieure à un intervenant faisant fonctionner le défibrillateur lorsque l'impédance avant choc du circuit de décharge de défibrillateur est supérieure à un seuil d'impédance de choc.

7. Produit programme informatique selon la revendication 6, dans lequel, en réponse à la décision d'administration de choc, le produit programme informatique comporte en outre des instructions pour : communiquer la décision d'administration de choc à l'intervenant faisant fonctionner le défibrillateur lorsque l'impédance avant choc du circuit de décharge de défibrillateur est inférieure au seuil d'impédance de choc.

8. Produit programme informatique selon la revendication 7,
dans lequel, en réponse à un choc administré par le défibrillateur, le produit programme informatique comporte en outre des instructions pour :
mesurer une impédance après choc du circuit de décharge de défibrillateur ; et
dans lequel, en réponse à une seconde décision d'administration de choc par l'analyseur ECG consécutive à l'administration d'un choc par le défibrillateur, le produit programme informatique comporte en outre des instructions pour :
mesurer une impédance hors choc d'un circuit de décharge de défibrillateur ;
estimer une seconde impédance avant choc du circuit de décharge de défibrillateur à partir d'une mesure de la seconde impédance hors choc du circuit de décharge de défibrillateur et d'une mesure de l'impédance après choc du circuit de décharge de défibrillateur ; et communiquer un second avis d'impédance de choc inférieure à un intervenant faisant fonctionner le défibrillateur lorsque la seconde impédance avant choc du défibrillateur du circuit de décharge de défibrillateur est supérieure au seuil d'impédance de choc.

9. Produit programme informatique selon la revendication 6, dans lequel, en réponse à une seconde décision d'administration de choc par un analyseur ECG à la suite de l'administration de choc par le défibrillateur, le produit programme informatique comprend en outre des instructions pour :
communiquer la seconde décision d'administration de choc à l'intervenant faisant fonctionner le défibrillateur lorsque la seconde impédance avant choc du circuit de décharge de défibrillateur est inférieure au seuil d'impédance de choc.

10. Produit programme informatique selon la revendication 6, dans lequel la décision d'administration de choc est pour une défibrillation sinusoïdale monophasique du cœur du patient et/ou une forme d'onde tronquée biphasique du cœur du patient.

11. Défibrillateur (40b), comprenant :
un moniteur ECG (50) configuré pour générer une forme d'onde ECG du cœur (11) d'un patient (10a) ; et
un dispositif de commande de défibrillateur (70) selon l'une quelconque des revendications 1 à 5.
